(19) Europäisches Patentamt
European Patent Office
Office européen des brevets

(11) EP 1 974 045 B1

(12) **EUROPÄISCHE PATENTSCHRIFT**

(45) Veröffentlichungstag und Bekanntmachung des Hinweises auf die Patenterteilung:
**15.06.2011 Patentblatt 2011/24**

(21) Anmeldenummer: **06841443.2**

(22) Anmeldetag: **19.12.2006**

(51) Int Cl.:
*C12P 7/24* (2006.01) *C12N 9/02* (2006.01)

(86) Internationale Anmeldenummer:
**PCT/EP2006/069894**

(87) Internationale Veröffentlichungsnummer:
**WO 2007/077121 (12.07.2007 Gazette 2007/28)**

(54) **VERFAHREN ZUR ENZYMATISCHEN HERSTELLUNG VON CITRONELLAL**

PROCESS FOR THE ENZYMATIC PREPARATION OF CITRONELLAL

PROCEDE DE FABRICATION ENZYMATIQUE DE CITRONELLAL

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **30.12.2005 DE 102005063191**

(43) Veröffentlichungstag der Anmeldung:
**01.10.2008 Patentblatt 2008/40**

(73) Patentinhaber: **BASF SE**
**67056 Ludwigshafen (DE)**

(72) Erfinder:
- **SCHÄDLER, Andreas**
**76835 Weyher (DE)**
- **FRIEDRICH, Thomas**
**64283 Darmstadt (DE)**
- **STÜRMER, Rainer**
**67127 Rödersheim-Gronau (DE)**
- **RINCK-JAHNKE, Sabine**
**69120 Heidelberg (DE)**

(74) Vertreter: **Popp, Andreas et al**
**BASF SE**
**Global Intellectual Property**
**GVX - C6**
**67056 Ludwigshafen (DE)**

(56) Entgegenhaltungen:
**EP-A- 1 557 459**

- **HALL M ET AL: "ASYMMETRIC WHOLE-CELL BIOREDUCTION OF AN ÄALPHAÜ,ÄBETAÜ-UNSATURATED ALDEHYDE (CITRAL): COMPETING PRIM-ALCOHOL DEHYDROGENASE AND C-C LYASE ACTIVITIES" TETRAHEDRON: ASYMMETRY, ELSEVIER SCIENCE PUBLISHERS, AMSTERDAM, NL, Bd. 17, Nr. 21, 17. November 2006 (2006-11-17), Seiten 3058-3062, XP008075048 ISSN: 0957-4166**
- **MULLER ET AL: "Enzymatic reduction of the alpha, beta-unsaturated carbon bond in citral" JOURNAL OF MOLECULAR CATALYSIS. B, ENZYMATIC, ELSEVIER, AMSTERDAM,, NL, Bd. 38, Nr. 3-6, 15. März 2006 (2006-03-15), Seiten 126-130, XP005309486 ISSN: 1381-1177**
- **WILLIAMS RICHARD E ET AL: "'New uses for an Old Enzyme': The Old Yellow Enzyme family of flavoenzymes" MICROBIOLOGY, SOCIETY FOR GENERAL MICROBIOLOGY, READING, GB, Bd. 148, Nr. 6, Juni 2002 (2002-06), Seiten 1607-1614, XP002419724 ISSN: 1350-0872**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 974 045 B1

## Beschreibung

Die Erfindung betrifft ein enzymatisches Verfahren zur Herstellung von optisch aktiven gesättigten Aldehyden oder Alkoholen der Formel aus α,β- ungesättigten Aldehyden, insbesondere ein Verfahren zur Herstellung von Citronellal.

Stand der Technik

(R)-(+) Citronellal ist ein wichtiges chemisches Zwischenprodukt, das beispielsweise bei der Herstellung von Menthol eingesetzt wird.

Die chemischen Methoden zur Herstellung von (R)-(+) Citronellal ausgehend von Citral erfordern sehr aufwändige Methoden (Ausschluß von Sauerstoff und Wasser), die bei einer technischen Synthese zu hohen Kosten führen.

Aufgabenstellung

Es bestand die Aufgabe, ein Verfahren zur Herstellung von Citronellal durch enantioselektive Reduktion von Citral bereitzustellen, das in einer hohen chemischen Ausbeute Citronellal möglichst enantiomerenrein liefert.

Beschreibung der Erfindung

Die vorliegende Erfindung betrifft ein Verfahren zur Herstellung von optisch aktiven gesättigten Aldehyden oder Alkoholen der Formel (2) aus α,β- ungesättigten Aldehyden der Formel (1) durch Reduktion in Gegenwart einer Enoat-Reduktase

(i) mit der Polypeptidsequenz SEQ ID NO:1 oder 2, oder
(ii) mit einer Polypeptidsequenz,die mindestens 80% Sequenzidentität mit SEQ ID NO:1 oder 2 aufweist.

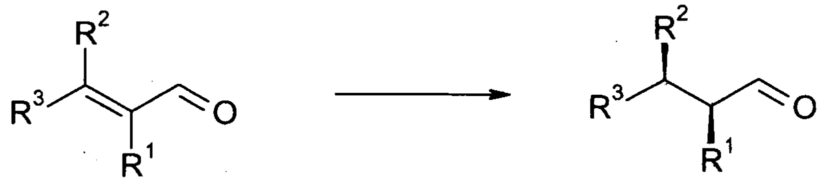

(1) (2)

wobei R1 und R2 unabhängig voneinander die Bedeutung H, $C_1$-$C_4$- Alkyl, R3 die Bedeutung H, $C_1$-$C_6$- Alkyl oder Alkenyl bedeuten in verzweigter und unverzweigter Form besitzen.

Das erfindungsgemäße Verfahren kann mit α,β- ungesättigten Aldehyden der Formel (1) durchgeführt werden, in denen R1 und R2 unabhängig voneinander die Bedeutung H, $C_1$-$C_4$- Alkyl in verzweigter und unverzweigter Form, R3 die Bedeutung H, $C_1$-$C_6$-Alkyl oder Alkenyl in verzweigter und unverzweigter Form besitzen. Die Alkyl- bzw. Alkenylreste können auch ein- oder mehrfach substituiert sein.

Besonders geeignete Substrate für das erfindungsgemäße Verfahren sind solche α,β-ungesättigten Aldehyden der Formel (1), bei denen R1 die Bedeutung H, R2 die Bedeutung $CH_3$ und R3 die Bedeutung $-CH_2-CH_2-CH=(CH_3)_2$ besitzen (Citral in cis oder trans Form) und solche, bei denen R1 die Bedeutung $CH_3$, R2 die Bedeutung H und R3 die Bedeutung $CH_3$ besitzen (2-Methyl-pent-2-en-1-al).

Die erfindungsgemäß verwendeten Enoat-Reduktasen reduzieren gelegentlich teilweise neben der Doppelbindung in α,β-Position zur Carbonylfunktion auch die Carbonylfunktion selbst, wodurch dann der entsprechende Alkohol gebildet wird.

Für das erfindungsgemässe Verfahren geeignete Enoat-Reduktasen sind solche Enzyme, die in der Lage sind, 2-Methyl-pent-2-en-1-al in einer NADPH abhängigen Reaktion zu (S)-2-Methyl-pentan-1-al zu reduzieren. Diese Reaktion wird im Folgenden auch Modellreaktion genannt.

Weiterhin besitzen die für das erfindungsgemässe Verfahren geeigneten Enoat-Reduktasen eine Polypeptidsequenz gemäss SEQ ID NO:1 oder NO:2 oder eine Polypeptidsequenz, die mindestens 80%, bevorzugt mindestens

90%, besonders bevorzugt mindestens 95% und insbesondere mindestens 97%, 98% oder 99% Sequenzidentität mit SEQ ID NO:1 oder 2 aufweist.

Ein Polypeptid mit der SEQ ID NO:1 ist das OYE2 Gen aus Bäckerhefe (Saccharomyces cerevisiae Genlocus YHR179W).

Ein Polypeptid mit der SEQ ID NO:2 ist das OYE3 Gen aus Bäckerhefe (Saccharomyces cerevisiae Genlocus YPL171C).

Die Ermittlung der Sequenzidentität soll für die hier beschriebenen Zwecke durch das Computerprogramm "GAP" der Genetics Computer Group (GCG) der University of Wisconsin erfolgen, wobei die Version 10.3 unter Verwendung der von GCG empfohlenen Standardparameter zum Einsatz kommen soll.

Solche Enoat-Reduktasen können ausgehend von SEQ ID NO:1 oder 2 durch dem Fachmann bekannte gezielte oder randomisierte Mutageneseverfahren erhalten werden. Alternativ kann jedoch auch in Mikroorganismen , bevorzugt in solchen der Gattungen *Alishewanella, Alterococcus, Aquamonas, Aranicola, Arsenophonus, Azotivirga, Brenneria, Buchnera (aphid P-endosymbionts), Budvicia, Buttiauxella, Candidatus Phlomobacter, Cedecea, Citrobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Pectobacterium, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia* oder *Yokenella,* nach Enoat-Reduktasen gesucht werden, die die o.g. Modellreaktion katalysieren und deren Aminosäuresequenz die geforderte Sequenzidentität zu SEQ ID NO:1 oder 2 bereits aufweist oder durch Mutageneseverfahren erhalten wird.

Die Enoat-Reduktase kann in gereinigter oder teilweise gereinigter Form oder auch in Form des Mikroorganismus selbst verwendet werden. Verfahren zur Gewinnung und Aufreinigung von Dehydrogenasen aus Mikroorganismen sind dem Fachmann hinreichend bekannt.

Bevorzugt erfolgt die die enantioselektive Reduktion mit der Enoat-Reduktase in Gegenwart eines geeigneten Cofaktors (auch als Cosubstrat bezeichnet). Als Cofaktoren für die Reduktion des Ketons dient üblicherweise NADH und/oder NADPH. Daneben können Enoat-Reduktasen als zelluläre Systeme eingesetzt werden, die inherent Cofaktor enthalten, oder alternative Redoxmediatoren zugesetzt werden (A. Schmidt, F. Hollmann und B. Bühler "Oxidation of Alcohols" in K. Drauz und H. Waldmann, Enzyme Catalysis in Organic Synthesis 2002, Vol.III, 991-1032, Wiley-VCH, Weinheim).

Bevorzugt erfolgt die enantioselektive Reduktion mit der Enoat-Reduktase außerdem in Gegenwart eines geeigneten Reduktionsmittels, welches den im Verlauf der Reduktion oxidierten Cofaktor regeneriert. Beispiele für geeignete Reduktionsmittels sind Zucker, insbesondere Hexosen, wie Glucose, Mannose, Fructose, und/oder oxidierbare Alkohole, insbesondere Ethanol, Propanol oder Isopropanol, sowie Formiat, Phosphit oder molekularer Wasserstoff. Zur Oxidation des Reduktionsmittels und damit verbunden zur Regeneration des Coenzyms kann eine zweite Dehydrogenase, wie z.B. Glucosedehydrogenase bei Verwendung von Glucose als Reduktionsmittel oder Formiat-Dehydrogenase bei der Verwendung von Formiat als Reduktionsmittel, zugesetzt werden. Diese kann als freies oder immobilisiertes Enzym oder in Form von freien oder immobilisierten Zellen eingesetzt werden. Ihre Herstellung kann sowohl separat als auch durch Coexpression in einem (rekombinanten) Reduktase-Stamm erfolgen.

Eine bevorzugte Ausführungsform des beanspruchten Verfahrens ist die Regenerierung der Cofaktoren durch ein enzymatisches System, bei dem eine zweite Dehydrogenase, besonders bevorzugt eine Glucosedehydrogenase, verwendet wird.

Ein weiterer Gegenstand der Erfindung ist die Verwendung der Enoat-Reduktase zur Herstellung von Citronellal.

Bei dieser Ausführungsform werden bevorzugt $NAD^+$ bzw. $NADP^+$ als Cofaktoren verwendet, die mit entsprechenden Cosubstaten (Oxidationsmitteln) wieder regeneriert werden können. Als Cosubstrat kann hier bevorzugt Aceton eingesetzt werden, das mit der bereits vorhandenen ADH und/oder einer zusätzlich verwendeten Dehydrogenase den Cofaktor regeneriert, wobei es zum Isopropanol reduziert wird.

"Enantioselektivität" im Rahmen der vorliegenden Erfindung bedeutet, dass der Enantiomerenüberschuss ee (in %) des S-Enantiomers, der sich in bekannter Weise berechnet nach:

$$ee\ (\%) = \text{S-Enantiomer} - \text{R-Enantiomer} / (\text{S-Enantiomer} - \text{R-Enantiomer}) \times 100$$

wenigstens 80 %, vorzugsweise wenigstens 90 %, insbesondere wenigstens 95 % und speziell wenigstens 97 % beträgt.

Die erfindungsgemäß verwendeten Enoat-Reduktasen können frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Crosslinking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol.III, 991-1032, Wiley-VCH, Weinheim beschrieben.

Die Umsetzung kann in wässrigen oder nichtwässrigen Reaktionsmedien oder in 2-Phasensystemen oder (Mikro-)Emulsionen erfolgen. Bei den wässrigen Reaktionsmedien handelt es sich vorzugsweise um gepufferte Lösungen, die in der Regel einen pH-Wert von 4 bis 8, vorzugsweise von 5 bis 8, aufweisen. Das wässrige Lösungsmittel kann neben Wasser außerdem wenigstens einen Alkohol, z.B. Ethanol oder Isopropanol oder Dimethylsulfoxid enthalten.

Unter nicht-wässrigen Reaktionsmedien werden Reaktionsmedien verstanden, die weniger als 1 Gew.-%, vorzugsweise weniger als 0,5 Gew.-% Wasser, bezogen auf das Gesamtgewicht des Reaktionsmediums, enthalten. Vorzugsweise wird die Umsetzung in einem organischen Lösungsmittel durchgeführt.

Geeignete Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Ether, insbesondere Tetrahydrofuran, verwendet.

Bevorzugt wird die Reduktion mit der Enoat-Reduktase in einem wässrig-organischen, insbesondere wässrigen Reaktionsmedium durchgeführt.

Das Substrat (1) wird vorzugsweise in einer Konzentration von 0,1 g/l bis 500 g/l, besonders bevorzugt von 1 g/l bis 50 g/l in die enzymatische Reduktion eingesetzt und kann kontinuierlich oder diskontinuierlich nachgeführt werden.

Die enzymatische Reduktion erfolgt in der Regel bei einer Reaktionstemperatur unterhalb der Desaktivierungstemperatur der eingesetzten Reduktase und oberhalb von -10 °C. Besonders bevorzugt liegt sie im Bereich von 0 bis 100 °C, insbesondere von 15 bis 60 °C und speziell von 20 bis 40 °C, z.B. bei etwa 30°C.

Zur Durchführung kann man beispielsweise das Substrat (1) mit der Enoat-Reduktase, dem Lösungsmittel und gegebenenfalls den Coenzymen, gegebenenfalls einer zweiten Dehydrogenase zur Regenerierung des Coenzyms und/ oder weiteren Reduktionsmitteln vorlegen und das Gemisch durchmischen, z. B. durch Rühren oder Schütteln. Es ist aber auch möglich, die Reduktase in einem Reaktor, beispielsweise in einer Säule, zu immobilisieren, und durch den Reaktor eine das Substrat und gegebenenfalls Coenzyme und/oder Cosubstrate enthaltende Mischung zu leiten. Hierzu kann man die Mischung im Kreislauf durch den Reaktor leiten bis der gewünschte Umsatz erreicht ist.

Dabei wird die Doppelbindung in $\alpha,\beta$-Position zur Carbonylfunktion zur Einfachbindung reduziert; gelegentlich erfolgt auch eine Reduktion der Carbonylfunktion selbst zur Alkoholfunktion. In der Regel wird man die Reduktion bis zu einem Umsatz von wenigstens 70 %, besonders bevorzugt von wenigstens 85 % und insbesondere von wenigstens 95%, bezogen auf das in der Mischung enthaltene Substrat führen. Das Fortschreiten der Reaktion, d. h. die sequentielle Reduktion der Doppelbindung kann dabei durch übliche Methoden wie Gaschromatographie oder Hochdruckflüssigkeitschromatographie verfolgt werden.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die die Modellreaktion katalysieren und die mindestens 20 %, bevorzugt 50 %, besonders bevorzugt 75 %, ganz besonders bevorzugt 90 % der Aktivität eines Enzyms, umfassend eine der unter SEQ ID NO:1 oder 2 aufgeführten Aminosäuresequenzen, aufweist. Funktionale Äquivalente sind außerdem vorzugsweise zwischen pH 4 bis 10 stabil und besitzen vorteilhaft ein pH-Optimum zwischen pH 5 und 8 sowie ein Temperaturoptimum im Bereich von 20°C bis 80°C.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gin ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate".

"Präkursoren" sind dabei natürliche oder synthetische Vorstufen der Polypeptide mit oder ohne der gewünschten biologischen Aktiviät.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Homologe des erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann

bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Gegenstand der Erfindung sind weiterhin Nukleinsäuresequenzen (einzel- und doppelsträngige DNA- und RNA-Sequenzen, wie z.B. cDNA und mRNA), die für ein Enzym mit erfindungsgemäßer Reduktase -Aktivität kodieren. Bevorzugt sind Nukleinsäuresequenzen, welche z.B. für Aminosäuresequenzen gemäß SEQ ID NO:1 oder 2 oder charakteristische Teilsequenzen davon kodieren.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Weitere Ausgestaltungen zur Durchführung des erfindungsgemäßen enzymatischen Reduktionsverfahrens

Die Enoat-Reduktasen können im erfindungsgemäßen Verfahren als freies oder immobilisiertes Enzym verwendet werden.

Das erfindungsgemäße Verfahren wird vorteilhaft bei einer Temperatur zwischen 0 °C bis 95 °C, bevorzugt zwischen 10 °C bis 85 °C, besonders bevorzugt zwischen 15 °C bis 75 °C durchgeführt.

Der pH-Wert im erfindungsgemäßen Verfahren wird vorteilhaft zwischen pH 4 und 12, bevorzugt zwischen pH 4,5 und 9, besonders bevorzugt zwischen -pH 5 und 8 gehalten. Unter enantiomerenreinen bzw. chiralen Produkten (2) sind im erfindungsgemäßen Verfahren Enantiomere zu verstehen, die eine Enantiomerenanreicherung zeigen. Bevorzugt werden im Verfahren Enantiomerenreinheiten von mindestens 70 %ee, bevorzugt von min. 80 %ee, besonders bevorzugt von min. 90 %ee, ganz besonders bevorzugt min. 98 %ee erreicht.

Für das erfindungsgemäße Verfahren können wachsende Zellen verwendet werden, die die erfindungsgemäßen Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die nachfolgenden Beispiele sollen die Erfindung veranschaulichen, ohne sie jedoch einzuschränken. Hierbei wird auf beiliegende Abbildungen Bezug genommen, dabei zeigt:

## Experimenteller Teil

### Biotransformation von 2-Methyl-pent-2-en-1-al mit Saccharomyces cerevisiae

Die Biotransformationen wurden in 500 ml Erlenmeyerkolben mit Schikanen durchgeführt. Es wurden 126 ml Transformationslösung vorgelegt. Zu den Ansätzen wurden 21 g D-Glucose zugegeben. Es wurde der gewünschte pH-Wert (zwischen 6 und 8,5) eingestellt. Die Substratmenge betrug pro Kolben 200 mg 2-Methyl-pent-2-en-1-al. Zum Startzeitpunkt wurden 21 g Bäckerhefe dazugegeben und die Ansätze bei der gewünschten Temperatur (zwischen 28 und 37°C) in den Inkubationsschrank gestellt (mit 240 rpm geschüttelt). Nach 6, 12, 18, 24, 36, 48 Stunden wurden Proben entnommen und gaschromatographisch analysiert.

Die höchsten Umsatzraten wurden bei pH-Werten zwischen 7,5 und 8,5 erreicht (40-70%). Die optische Reinheit betrug bei pH= 8,5 und T=37°C: ee=92,1 bei einem Umsatz von 66,6%.

Biotransformation von Citral mit Saccharomyces cerevisiae

Die Biotransformationen wurden in 500 ml Erlenmeyerkolben mit Schikanen durchgeführt. Es wurden 126 ml Transformationslösung vorgelegt. Zu den Ansätzen wurden 21 g D-Glucose zugegeben. Es wurde der gewünschte pH-Wert (zwischen 6 und 8,5) eingestellt. Die Substratmenge betrug pro Kolben 210 mg Citral. (Citral war ein cis/trans Gemisch von 70:30) Zum Startzeitpunkt wurden 21 g Bäckerhefe dazugegeben und die Ansätze bei der gewünschten Temperatur (zwischen 28 und 37°C) in den Inkubationsschrahk gestellt (mit 240 rpm geschüttelt). Nach 6, 12, 18, 24, 36, 48 Stunden wurden Proben entnommen und gaschromatographisch analysiert.
Man erhielt neben (R)-(+)-Citronellal auch noch (R)-(+)-ß-Citronellol sowie Nerol und Geraniol.

Transformationslösung

53,4 g Na2HPO4 und 21 g D-Glucose und 126 ml Wasser dest. (pH eingestellt zwischen 6 und 8,5).

SEQUENCE LISTING

<110> BASF Aktiengesellschaft

<120> Verfahren zur enzymatischen Herstellung von Citronellal

<130> AE20040629

<160> 2

<170> Patent In version 3. 1

<210> 1

<211> 400

<212> PRT

<213> Saccharomyces cerevisiae

<400> 1

Met Pro Phe Val Lys Asp Phe Lys Pro Gln Ala Leu Gly Asp Thr Asn
1 5 10 15

Leu Phe Lys Pro Ile Lys Ile Gly Asn Asn Glu Leu Leu His Arg Ala
20 25 30

Val Ile Pro Pro Leu Thr Arg Met Arg Ala Gln His Pro Gly Asn Ile
35 40 45

Pro Asn Arg Asp Trp Ala Val Glu Tyr Tyr Ala Gln Arg Ala Gln Arg
50 55 60

Pro Gly Thr Leu Ile Ile Thr Glu Gly Thr Phe Pro Ser Pro Gln Ser
65 70 75 80

Gly Gly Tyr Asp Asn Ala Pro Gly Ile Trp Ser Glu Glu Gln Ile Lys
85 90 95

Glu Trp Thr Lys Ile Phe Lys Ala Ile His Glu Asn Lys Ser Phe Ala
100 105 110

Trp Val Gln Leu Trp Val Leu Gly Trp Ala Ala Phe Pro Asp Thr Leu
115 120 125

Ala Arg Asp Gly Leu Arg Tyr Asp Ser Ala Ser Asp Asn Val Tyr Met
130 135 140

Asn Ala Glu Gln Glu Glu Lys Ala Lys Lys Ala Asn Asn Pro Gln His
145 150 155 160

Ser Ile Thr Lys Asp Glu Ile Lys Gln Tyr Val Lys Glu Tyr Val Gln
165 170 175

Ala Ala Lys Asn Ser Ile Ala Ala Gly Ala Asp Gly Val Glu Ile His
180 185 190

Ser Ala Asn Gly Tyr Leu Leu Asn Gln Phe Leu Asp Pro His Ser Asn
195 200 205

Asn Arg Thr Asp Glu Tyr Gly Gly Ser Ile Glu Asn Arg Ala Arg Phe
210 215 220

Thr Leu Glu Val Val Asp Ala Val Val Asp Ala Ile Gly Pro Glu Lys
225 230 235 240

Val Gly Leu Arg Leu Ser Pro Tyr Gly Val Phe Asn Ser Met Ser Gly
245 250 255

Gly Ala Glu Thr Gly Ile Val Ala Gln Tyr Ala Tyr Val Leu Gly Glu
260 265 270

Leu Glu Arg Arg Ala Lys Ala Gly Lys Arg Leu Ala Phe Val His Leu
275 280 285

Val Glu Pro Arg Val Thr Asn Pro Phe Leu Thr Glu Gly Glu Gly Glu
290 295 300

Tyr Asn Gly Gly Ser Asn Lys Phe Ala Tyr Ser Ile Trp Lys Gly Pro
305 310 315 320

Ile Ile Arg Ala Gly Asn Phe Ala Leu His Pro Glu Val Val Arg Glu
325 330 335

Glu Val Lys Asp Pro Arg Thr Leu Ile Gly Tyr Gly Arg Phe Phe Ile
340 345 350

Ser Asn Pro Asp Leu Val Asp Arg Leu Glu Lys Gly Leu Pro Leu Asn
355 360 365

Lys Tyr Asp Arg Asp Thr Phe Tyr Lys Met Ser Ala Glu Gly Tyr Ile
370 375 380

Asp Tyr Pro Thr Tyr Glu Glu Ala Leu Lys Leu Gly Trp Asp Lys Asn
385 390 395 400

<210> 2<211> 400<212> PRT<213> Saccharomyces cerevisiae<400> 2

Met Pro Phe Val Lys Gly Phe Glu Pro Ile Ser Leu Arg Asp Thr Asn
1 5 10 15

Leu Phe Glu Pro Ile Lys Ile Gly Asn Thr Gln Leu Ala His Arg Ala
20 25 30

Val Met Pro Pro Leu Thr Arg Met Arg Ala Thr His Pro Gly Asn Ile
35 40 45

Pro Asn Lys Glu Trp Ala Ala Val Tyr Tyr Gly Gln Arg Ala Gln Arg

50 55 60

Pro Gly Thr Met Ile Ile Thr Glu Gly Thr Phe Ile Ser Pro Gln Ala
65 70 75 80

Gly Gly Tyr Asp Asn Ala Pro Gly Ile Trp Ser Asp Glu Gln Val Ala
85 90 95

Glu Trp Lys Asn Ile Phe Leu Ala Ile His Asp Cys Gln Ser Phe Ala
100 105 110

Trp Val Gln Leu Trp Ser Leu Gly Trp Ala Ser Phe Pro Asp Val Leu
115 120 125

Ala Arg Asp Gly Leu Arg Tyr Asp Cys Ala Ser Asp Arg Val Tyr Met
130 135 140

Asn Ala Thr Leu Gln Glu Lys Ala Lys Asp Ala Asn Asn Leu Glu His
145 150 155 160

Ser Leu Thr Lys Asp Asp Ile Lys Gln Tyr Ile Lys Asp Tyr Ile His
165 170 175

Ala Ala Lys Asn Ser Ile Ala Ala Gly Ala Asp Gly Val Glu Ile His
180 185 190

Ser Ala Asn Gly Tyr Leu Leu Asn Gln Phe Leu Asp Pro His Ser Asn
195 200 205

Lys Arg Thr Asp Glu Tyr Gly Gly Thr Ile Glu Asn Arg Ala Arg Phe
210 215 220

Thr Leu Glu Val Val Asp Ala Leu Ile Glu Thr Ile Gly Pro Glu Arg
225 230 235 240

Val Gly Leu Arg Leu Ser Pro Tyr Gly Thr Phe Asn Ser Met Ser Gly
245 250 255

Gly Ala Glu Pro Gly Ile Ile Ala Gln Tyr Ser Tyr Val Leu Gly Glu
260 265 270

Leu Glu Lys Arg Ala Lys Ala Gly Lys Arg Leu Ala Phe Val His Leu
275 280 285

Val Glu Pro Arg Val Thr Asp Pro Ser Leu Val Glu Gly Glu Gly Glu
290 295 300

Tyr Ser Glu Gly Thr Asn Asp Phe Ala Tyr Ser Ile Trp Lys Gly Pro
305 310 315 320

Ile Ile Arg Ala Gly Asn Tyr Ala Leu His Pro Glu Val Val Arg Glu
325 330 335

| | | | | | | | | | | | | | | | |
|---|---|---|---|---|---|---|---|---|---|---|---|---|---|---|---|
| Gln | Val | Lys | Asp<br>340 | Pro | Arg | Thr | Leu | Ile<br>345 | Gly | Tyr | Gly | Arg | Phe<br>350 | Phe | Ile |
| Ser | Asn | Pro<br>355 | Asp | Leu | Val | Tyr | Arg<br>360 | Leu | Glu | Glu | Gly | Leu<br>365 | Pro | Leu | Asn |
| Lys | Tyr<br>370 | Asp | Arg | Ser | Thr | Phe<br>375 | Tyr | Thr | Met | Ser | Ala<br>380 | Glu | Gly | Tyr | Thr |
| Asp<br>385 | Tyr | Pro | Thr | Tyr | Glu<br>390 | Glu | Ala | Val | Asp | Leu<br>395 | Gly | Trp | Asn | Lys | Asn<br>400 |

**Patentansprüche**

1. Verfahren zur Herstellung von optisch aktiven gesättigten Aldehyden oder Alkoholen der Formel (2) aus α,β- ungesättigten Aldehyden der Formel (1) durch Reduktion in Gegenwart einer Enoat-Reduktase

   (i) mit der Polypeptidsequenz SEQ ID NO: oder 2, oder
   (ii) mit einer Polypeptidsequenz, die mindestens 80% Sequenzidentität mit SEQ ID NO: 1 oder 2 aufweist

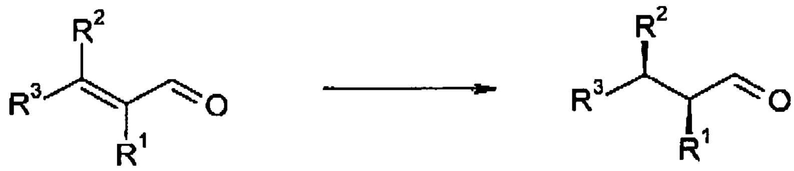

(1) (2)

   wobei R1 und R2 unabhängig voneinander die Bedeutung H, $C_1$-$C_4$-Alkyl, R3 die Bedeutung H, $C_1$-$C_6$- Alkyl oder Alkenyl in verzweigter und unverzweigter Form besitzen.

2. Verfahren nach Anspruch 1, **dadurch gekennzeichnet, dass** die Reduktion mit NADPH als Cofaktor ausgeführt wird.

3. Verfahren nach Anspruch 1, **dadurch gekennzeichnet, dass** der verwendete Cofaktor enzymatisch regeneriert wird.

4. Verfahren nach Anspruch 1, **dadurch gekennzeichnet, dass** die Cofaktor-Regenerierung durch Glucose-Dehydrogenase erfolgt.

5. Verfahren nach Anspruch 1, **dadurch gekennzeichnet, dass** die Reduktion in wässrigem System durchgeführt wird.

6. Verfahren nach Anspruch 1, **dadurch gekennzeichnet, dass** die Enoat-Reduktase immobilisiert vorliegt.

7. Verwendung einer Enoat-Reduktase

   (i) mit der Polypeptidsequenz SEQ ID NO:1 oder 2, oder

(ii) mit einer Polypeptidsequenz, die mindestens 80% Sequenzidentität mit SEQ ID NO:1 oder 2 aufweist,

in einem Verfahren zur Herstellung von (R)-(+)Citronellal aus Citral.

## Claims

1. A process for preparing optically active saturated aldehydes or alcohols of the formula (2) from a,β-unsaturated aldehydes of the formula (1) by reduction in the presence of an enoate reductase

(i) having the polypeptide sequence SEQ ID No.1 or 2, or
(ii) having a polypeptide sequence which is at least 80% identical to the sequence of SEQ ID No.1 or 2,

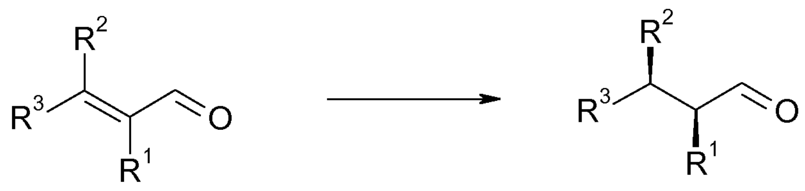

(1) (2)

in which R1 and R2 independently of one another are H, $C_1$-$C_4$-alkyl, R3 is H, $C_1$-$C_6$-alkyl or alkenyl, in branched and unbranched form.

2. The process according to claim 1, wherein the reduction is carried out using NADPH as cofactor.

3. The process according to claim 1, wherein the cofactor used is regenerated enzymatically.

4. The process according to claim 1, wherein the cofactor is regenerated by glucose dehydrogenase.

5. The process according to claim 1, wherein the reduction is carried out in an aqueous system.

6. The process according to claim 1, wherein the enoate reductase is present in an immobilized form.

7. The use of an enoate reductase

(i) having the polypeptide sequence SEQ ID No.1 or 2, or
(ii) having a polypeptide sequence which is at least 80% identical to the sequence of SEQ ID No.1 or 2,

in a process for preparing (R)-(+)citronellal from citral.

## Revendications

1. Procédé pour la préparation d'alcools ou d'aldéhydes saturés optiquement actifs de formule (2) à partir d'aldéhydes α,β-insaturés de formule (1) par réduction en présence d'une énoate réductase

(i) ayant la séquence de polypeptide SEQ ID n° 1 ou 2, ou
(ii) ayant une séquence de polypeptide qui présente une identité de séquence d'au moins 80 % avec SEQ ID n° 1 ou 2

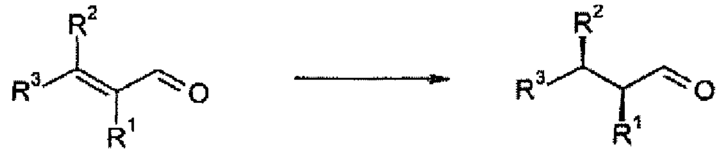

(1) (2)

R1 et R2 représentant indépendamment l'un de l'autre H, un groupe alkyle en $C_1$-$C_4$, R3 représentant H, un groupe alkyle ou alcényle en $C_1$-$C_6$ sous forme ramifiée ou non ramifiée.

2. Procédé selon la revendication 1, **caractérisé en ce que** la réduction est effectuée avec NADPH en tant que cofacteur.

3. Procédé selon la revendication 1, **caractérisé en ce que** le cofacteur utilisé est régénéré par voie enzymatique.

4. Procédé selon la revendication 1, **caractérisé en ce que** la régénération du cofacteur s'effectue à l'aide de glucose déshydrogénase.

5. Procédé selon la revendication 1, **caractérisé en ce que** la réduction est effectuée en système aqueux.

6. Procédé selon la revendication 1, **caractérisé en ce que** l'énoate réductase est présente immobilisée.

7. Utilisation d'une énoate réductase

   (i) ayant la séquence de polypeptide SEQ ID n° 1 ou 2, ou
   (ii) ayant une séquence de polypeptide qui présente une identité de séquence d'au moins 80 % avec SEQ ID n° 1 ou 2,

   dans un procédé pour la préparation de (R)-(+)-citronellal à partir de citral.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1149849 A
- EP 1069183 A
- DE OS100193773 A


### In der Beschreibung aufgeführte Nicht-Patentliteratur


- Oxidation of Alcohols. **A. Schmidt ; F. Hollmann ; B. Bühler ; K. Drauz ; H. Waldmann.** Enzyme Catalysis in Organic Synthesis. Wiley-VCH, 2002, vol. III, 991-1032
- Immobilization of Enzymes. **J. Lalonde ; A. Margolin ; K. Drauz ; H. Waldmann.** Enzyme Catalysis in Organic Synthesis. Wiley-VCH, 2002, vol. III, 991-1032
- **Narang, S.A.** *Tetrahedron,* 1983, vol. 39, 3
- **Itakura et al.** *Annu. Rev. Biochem.,* 1984, vol. 53, 323
- **Itakura et al.** *Science,* 1984, vol. 198, 1056
- **Ike et al.** *Nucleic Acids Res.,* 1983, vol. 11, 477
- **Arkin ; Yourvan.** *PNAS,* 1992, vol. 89, 7811-7815
- **Delgrave et al.** *Protein Engineering,* 1993, vol. 6 (3), 327-331
- Voet, Voet. Wiley Press, 896-897
- **Sambrook et al.** Molecular Cloning: A laboratory manual. Cold Spring Harbor Laboratory Press, 1989
- Biotechnology. 1993, vol. 3